# EUROPEAN PATENT APPLICATION

(11) **EP 4 293 261 A1**
(43) Date of publication of application: **20.12.2023**
(21) Application number: 22752875.9
(22) Date of filing: 15.02.2022
(51) Int. Cl.: F16K 31/126, F04B 43/04, A61B 5/0225

(54) **MEASURING DEVICE**

(30) Priority: 15.02.2021 JP 2021022108
(71) Applicant: Minebea Mitsumi Inc., Kitasaku-gun, Nagano 3890293 (JP)
(72) Inventor: KURITA, Daisuke, Kitasaku-gun, Nagano 389-0293 (JP); IIDA, Masahiro, Kitasaku-gun, Nagano 389-0293 (JP); IRIE, Takahiko, Tama-shi, Tokyo 206-8567 (JP); KODAMA, Daisuke, Tama-shi, Tokyo 206-8567 (JP); UEDA, Kenta, Tama-shi, Tokyo 206-8567 (JP); YOSHII, Yuta, Tama-shi, Tokyo 206-8567 (JP)
(74) Representative: Zabel, Julia Elisabeth
(86) International application number: PCT/JP2022/005863
(87) International publication number: WO 2022/173053

(57) **Abstract**

A measuring device having a first space for pressurizing an object to be pressurized and a second space independent of the first space. The measuring device is provided with a first exhaust valve that closes or opens a communication port for communication between the first space and another space. The open/closed state of the first exhaust valve changes depending on a pressure difference between the first space and the second space.

## Description

### Technical Field

The present invention relates to a measuring device.

### Background Art

In measuring devices such as a blood pressure monitor, a so-called pressure rising type measuring device is known, the pressure rising type measuring device measuring blood pressure during pressurization, not in a depressurization process after pressurization. In the pressure rising type measuring device, it is required to rapidly exhaust air pressurized in a cuff when the pressurization is stopped. For example, a measuring device using a solenoid valve for rapidly exhausting pressurized air is known.

### Citation List

### Patent Literature

Patent Document 1: JP 2018-171311 A

### Summary of Invention

### Technical Problem

Providing a solenoid valve complicates a structure of the measuring device, increases a manufacturing cost and power consumption, and hinders miniaturization of the measuring device.

In one aspect, an object is to provide a measuring device capable of simplifying a structure.

### Solution to Problem

In one aspect, a measuring device includes a first space for pressurizing a pressurization target and a second space independent of the first space. The measuring device includes a first exhaust valve configured to close or open a communication port communicating the first space with another space. An open/closed state of the first exhaust valve changes in accordance with a pressure difference between the first space and the second space.

According to one aspect, the structure of the measuring device can be simplified.

### Brief Description of Drawings

FIG. 1 is a perspective view illustrating an example of an overall configuration of a measuring device according to an embodiment.
FIG. 2 is a cross-sectional view illustrating an example of a pump in a first embodiment.
FIG. 3 is a cross-sectional view illustrating an example of a pressure regulating mechanism in the first embodiment.
FIG. 4 is a cross-sectional view illustrating an example of an intake state in the pressure regulating mechanism in the first embodiment.
FIG. 5 is a cross-sectional view illustrating an example of an exhaust state of the pressure regulating mechanism in the first embodiment.
FIG. 6 is a cross-sectional view illustrating an example of a pump according to a second embodiment.
FIG. 7 is a cross-sectional view illustrating an example of a pressure regulating mechanism in the second embodiment.
FIG. 8 is a cross-sectional view illustrating an example of an intake state in the pressure regulating mechanism in the second embodiment.
FIG. 9 is a cross-sectional view illustrating an example of an exhaust state of the pressure regulating mechanism in the second embodiment.
FIG. 10 is an enlarged cross-sectional view illustrating an example of a positional relationship between a first exhaust valve and an opening in a first modification.
FIG. 11 is an enlarged cross-sectional view illustrating an example of the operation of the first exhaust valve in the intake state according to the first modification.
FIG. 12 is an enlarged cross-sectional view illustrating another example of the operation of the first exhaust valve in the intake state according to the first modification.
FIG. 13 is an enlarged cross-sectional view illustrating an example of the operation of the first exhaust valve in the exhaust state according to the first modification.
FIG. 14 is an enlarged cross-sectional view illustrating another example of the operation of the first exhaust valve in the exhaust state in a second modification.

### Description of Embodiments

Hereinafter, an embodiment of a measuring device disclosed in the present application will be described in detail with reference to the drawings. Note that in the drawings, the dimensional relationship of elements and the ratio of the elements may differ from reality. Among the drawings, parts having mutually different dimensional relationships and proportions may be included. In each of the following embodiments and modifications, the same parts as those illustrated in the drawings described above are denoted by the same reference numerals, and redundant description will be omitted.

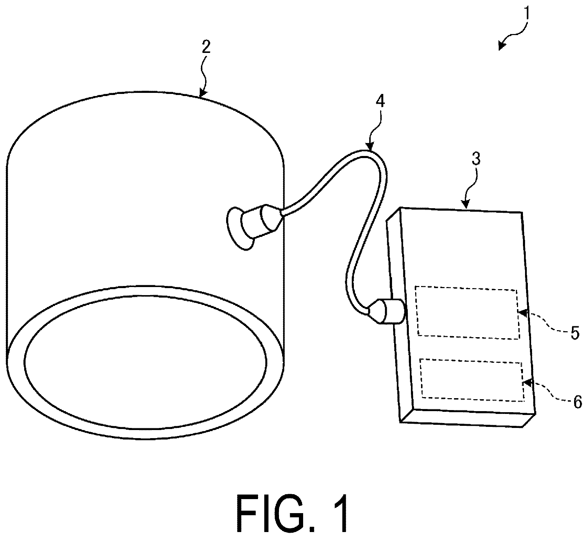

First, a measuring device according to each embodiment will be described with reference to FIG. 1. FIG. 1 is a perspective view illustrating an example of an overall configuration of a measuring device according to an embodiment. As illustrated in FIG. 1, a measuring device 1 in each embodiment is, for example, an electronic blood pressure monitor, and includes a cuff 2, a main body 3, and a tube 4 connecting the cuff 2 and the main body 3. The main body 3 includes a pump unit 5 and a control unit 6. The cuff 2 is an example of a pressurization target.

The pump unit 5 of the main body 3 supplies a fluid to the cuff 2 via the tube 4. In each embodiment, for example, air is supplied as the fluid. The control unit 6 of the main body 3 controls, for example, start and stop of the pump unit 5.

The cuff 2 is attached to a measurement site such as a wrist of a subject, for example. The cuff 2 pressurizes the measurement site with air supplied from the main body 3. In each of the embodiments, the air supplied to the cuff 2 is not exhausted except from the tube 4. The air supplied to the cuff 2 is exhausted through the tube 4 in accordance with the operation of the pump unit 5 described below.

### First Embodiment

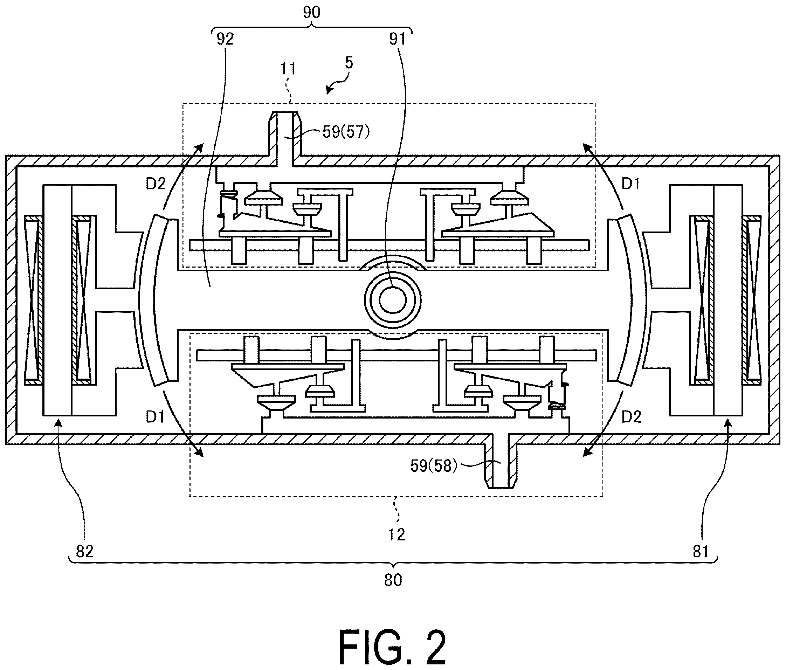

Next, the pump in the first embodiment will be described. FIG. 2 is a cross-sectional view illustrating an example of the pump according to the first embodiment. As illustrated in FIG. 2, the pump unit 5 includes a drive mechanism 80, a swing mechanism 90, a pair of pressure regulating mechanisms 11 and 12, and a pair of discharge ports 57 and 58. The number of pressure regulating mechanisms included in the pump unit 5 is not limited to two, and may be one or three or more. Hereinafter, the pair of pressure regulating mechanisms 11 and 12 may be referred to as a pressure regulating mechanism 10 when the pressure regulating mechanisms 11 and 12 are not distinguished from each other. In addition, the pair of discharge ports 57 and 58 may be referred to as a discharge port 59 when the discharge ports 57 and 58 are not distinguished from each other.

The drive mechanism 80 is an operation unit swinging the swing mechanism 90, and includes, for example, a pair of actuators 81 and 82. For example, the drive mechanism 80 starts or stops the operation based on a control signal transmitted from the control unit 6. The actuators 81 and 82 are, for example, vibration actuators using a solenoid, a motor, or the like. As the vibration actuator, a well-known actuator such as an actuator using a piezoelectric element or an actuator using resonance of an elastic body can be applied.

The swing mechanism 90 illustrated in FIG. 2 includes, for example, a shaft 91 and a swing body 92. The swing body 92 swings in the vertical direction in the drawing with the shaft 91 as a swing axis, thereby pressing a pressing portion of the pressure regulating mechanism 10 described below.

The pressure regulating mechanism 10 pressurizes the cuff 2 by supplying air to the cuff 2 via the discharge port 59 and the tube 4, and depressurizes the cuff 2 by exhausting the air supplied to the cuff 2. The pair of pressure regulating mechanisms 11 and 12 are both connected to the cuff 2 via the tube 4.

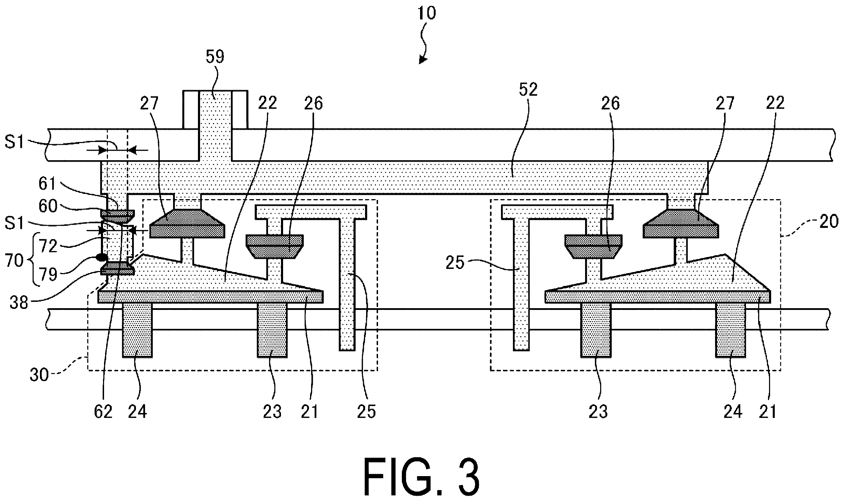

FIG. 3 is a cross-sectional view illustrating an example of the pressure regulating mechanism in the first embodiment. Note that FIG. 3 illustrates a state where neither pressurization nor depressurization is performed. As illustrated in FIG. 3, the pressure regulating mechanism 10 according to the first embodiment includes first pressurizing units 20 and 30, a first space 52, a first exhaust valve 60, and a pressure regulating unit 70. The two first pressurizing units 20 and 30 are connected to the same first space 52.

As illustrated in FIG. 3, the first pressurizing unit 20 includes a movable portion 21, a sealed space 22, pressing portions 23 and 24, an intake port 25, an intake valve 26, and a third exhaust valve 27. The movable portion 21 constitutes a part of the sealed space 22. The movable portion 21 changes the volume of the sealed space 22 by changing the position and inclination in conjunction with the pressing portions 23 and 24. The movable portion 21 is a diaphragm formed of a material having flexibility such as rubber.

The sealed space 22 repeats intake and exhaust in accordance with increase and decrease of the volume. When the volume of the sealed space 22 increases, air is supplied to the sealed space 22 via the intake port 25 and the intake valve 26. When the volume of the sealed space 22 decreases, air is supplied to the first space 52 via the third exhaust valve 27.

The pressing portions 23 and 24 displace the position and inclination of the movable portion 21 in conjunction with the swing body 92. For example, when the swing body 92 illustrated in FIG. 2 swings in the direction indicated by the arrow D1, the pressing portions 23 and 24 push up the movable portion 21 of the first pressurizing unit 20. As a result, the volume of the sealed space 22 decreases. When the swing body 92 illustrated in FIG. 2 swings in the direction indicated by the arrow D2, air is supplied to the sealed space 22 of the first pressurizing unit 20 via the intake port 25 and the intake valve 26. Accordingly, the pressing portions 23 and 24 are pressed down by the movable portion 21.

The intake port 25 connects the sealed space 22 to another space such as the outside of the device. The intake valve 26 is, for example, a check valve. The intake valve 26 allows a flow of air from the intake port 25 to the sealed space 22, and restricts a flow of air from the sealed space 22 to the intake port 25. The third exhaust valve 27 is provided between the sealed space 22 and the first space 52. The third exhaust valve 27 is also, for example, a check valve. The third exhaust valve 27 allows a flow of air from the sealed space 22 to the first space 52 and restricts a flow of air from the first space 52 to the sealed space 22. By the intake valve 26 and the third exhaust valve 27, air is supplied from the other space to the first space 52 via the sealed space 22.

As illustrated in FIG. 3, the first pressurizing unit 30 further includes a second exhaust valve 38 in addition to the movable portion 21, the sealed space 22, the pressing portions 23 and 24, the intake port 25, the intake valve 26, and the third exhaust valve 27. Similarly to the third exhaust valve 27 of the first pressurizing unit 20, the third exhaust valve 27 of the first pressurizing unit 30 is also provided between the sealed space 22 of the first pressurizing unit 30 and the first space 52.

In addition, the second exhaust valve 38 is provided between the sealed space 22 and a second space 72 of the pressure regulating unit 70 to be described below. The second exhaust valve 38 is also, for example, a check valve. The second exhaust valve 38 allows a flow of air from the sealed space 22 to the second space 72, and restricts a flow of air from the second space 72 to the sealed space 22. Thus, the first pressurizing unit 30 supplies air to the first space 52, and also supplies air to the second space 72.

As illustrated in FIG. 3, the first pressurizing unit 30 and the first pressurizing unit 20 in the first embodiment are disposed at positions facing each other, across the shaft 91, in the direction in which the swing body 92 extends. That is, when the swing body 92 illustrated in FIG. 2 swings in the direction indicated by the arrow D1, air is supplied to the sealed space 22 of the first pressurizing unit 20, while air is supplied from the sealed space 22 of the first pressurizing unit 30 to the first space 52. Conversely, when the swing body 92 illustrated in FIG. 2 swings in the direction indicated by the arrow D2, air is supplied from the sealed space 22 of the first pressurizing unit 20 to the first space 52, while air is supplied to the sealed space 22 of the first pressurizing unit 30. Thus, air is continuously supplied from the first pressurizing units 20 and 30 to the first space 52.

The discharge port 59 connects the first space 52 and the tube 4. The pair of discharge ports 57 and 58 are provided corresponding to the pair of pressure regulating mechanisms 11 and 12, respectively. That is, the first space 52 in the pressure regulating mechanism 11 is connected to the cuff 2 via the discharge port 57 and the tube 4. Similarly, the first space 52 in the pressure regulating mechanism 12 is connected to the cuff 2 via the discharge port 58 and the tube 4. As a result, air is supplied to the cuff 2 from both of the pair of pressure regulating mechanisms 11 and 12.

In the first embodiment, air is supplied to the first space 52 of the pressure regulating mechanism 10 from the first pressurizing unit 20 and the first pressurizing unit 30. In the first embodiment, the first space 52 and the cuff 2 constitute a sealed space when the first exhaust valve 60 described below is in a closed state. In this case, the air supplied to the first space 52 is supplied to the cuff 2 via the discharge port 59 and the tube 4, thereby pressurizing the cuff 2.

On the other hand, when the first exhaust valve 60 is in an open state, the air in the first space 52 is exhausted from the first exhaust valve 60 to the other space. At this time, air is also exhausted via the first exhaust valve 60 from the cuff 2 connected via the discharge port 59 and the tube 4. In the first embodiment, the air pressure in the first space 52 and the cuff 2 increases according to the pressurization by the first pressurizing unit 20 and the first pressurizing unit 30, with the air pressure in the other space as the lower limit. Hereinafter, the first exhaust valve 60 in the closed state may be referred to as a first exhaust valve 60c, and the first exhaust valve 60 in the open state may be referred to as a first exhaust valve 60o.

The pressure regulating unit 70 illustrated in FIG. 3 includes the second space 72 and an air leakage valve 79. The second space 72 is a space independent of the first space 52. The second space 72 is not a sealed space, but communicates with the other space via the air leakage valve 79, and air constantly leaks to the other space.

In the first embodiment, air is supplied to the second space 72 from the first pressurizing unit 30 illustrated in FIG. 3 via the second exhaust valve 38. Further, the amount of air supplied by the first pressurizing unit 30 is larger than the amount of air leaking from the air leakage valve 79. That is, in the first embodiment, the air pressure in the second space 72 increases according to the pressurization by the first pressurizing unit 30 with the air pressure in the other space as a lower limit. In this case, as will be described below, the second space 72 is pressurized by the first pressurizing unit 30, so that the air pressure inside the second space 72 becomes higher than the air pressure inside the first space 52.

Further, in the first embodiment, when the pressurization by the first pressurizing unit 30 is stopped, the air supplied into the second space 72 leaks from the air leakage valve 79 to the other space. As a result, the air pressure in the second space 72 rapidly approaches the air pressure in the other space. As a result, as will be described below, the air pressure inside the second space 72 becomes lower than the air pressure inside the first space 52.

The first exhaust valve 60 closes or opens the first space 52 and the other space such as the outside of the device. The first exhaust valve 60 is provided, for example, between the first space 52 and the second space 72. The first exhaust valve 60 is, for example, a check valve, and regulates the flow of air from the other space to the first space 52 and the flow of air from the second space 72 to the other space. For example, as illustrated in FIG. 3, the first exhaust valve 60 includes a first surface 61 facing the first space 52 and a second surface 62 facing the second space 72. The first exhaust valve 60 in the embodiment is, for example, a rapid exhaust valve. In the first embodiment, the pressure receiving area S1 of the first surface 61 with respect to the first space 52 and the pressure receiving area S1 with respect to the second space 72 are substantially the same.

In the first embodiment, for example, the open/closed state of the first exhaust valve 60 changes according to the air pressure difference (differential pressure) between the first space 52 and the second space 72. The first exhaust valve 60 changes between the open state 60o and the closed state 60c by, for example, changing position or changing shape, such as the size of an opening, in accordance with the differential pressure between the first space 52 and the second space 72.

For example, the first exhaust valve 60 is in the closed state when the air pressure in the second space 72 is equal to or higher than the air pressure in the first space 52. In this case, since the first space 52 is closed with respect to the other space, the air in the first space 52 is exhausted from the discharge port 59 to the cuff 2.

On the other hand, for example, the first exhaust valve 60 is in the open state when the air pressure in the second space 72 is lower than the air pressure in the first space 52. In this case, the air in the first space 52 is exhausted to the other space via the first exhaust valve 60, whereby the air pressure in the first space 52 rapidly approaches the air pressure in the other space. Along with this, the air supplied from the first space 52 to the cuff 2 is also sucked into the first space 52, so the air pressure in the cuff 2 also decreases rapidly.

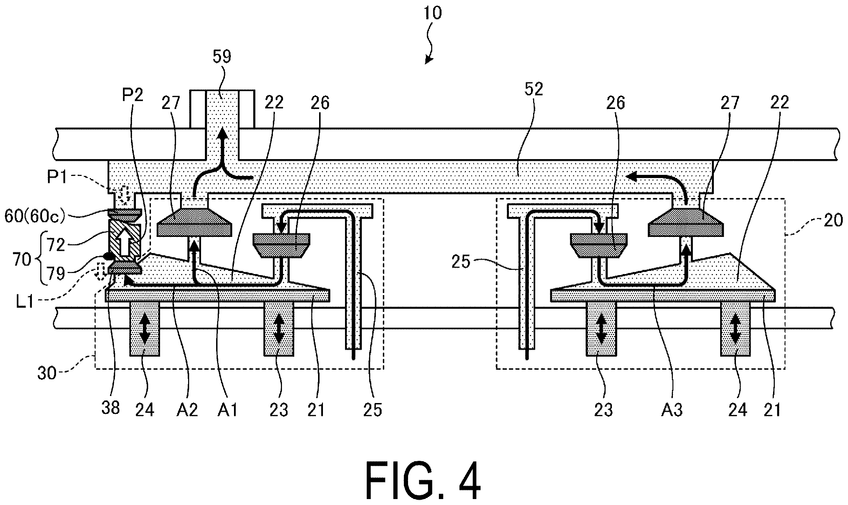

As illustrated in FIG. 3, the volume of the second space 72 is much smaller than the volume of the first space 52. In this case, for example, when the first pressurizing unit 30 pressurizes both the first space 52 and the second space 72 as illustrated in FIG. 4, the increase of the air pressure in the second space 72 is faster than the increase of the air pressure in the first space 52. In this case, as illustrated in FIG. 4, an intake state such that the first exhaust valve 60 is closed is achieved. FIG. 4 is a cross-sectional view illustrating an example of the intake state in the pressure regulating mechanism according to the first embodiment. As illustrated in FIG. 4, in the intake state, the pressing portions 23 and 24 move up and down in the directions indicated by the arrows, so that air is supplied from the first pressurizing units 20 and 30 to the first space 52 as indicated by arrows A1 and A3. Similarly, as indicated by the arrow A2, air is also supplied from the first pressurizing unit 30 to the second space 72.

In this case, the amount of air illustrated by the arrow A2 supplied to the second space 72 is larger than the amount of air illustrated by the arrow L1 leaking from the air leakage valve 79. The volume of the second space 72 is much smaller than the volume of the first space 52. For this reason, when the first pressurizing unit 30 starts pressurization, the air pressure P2 in the second space 72 rapidly increases compared to the increase in the air pressure P1 in the first space 52. As a result, the air pressure P2 in the second space 72 becomes higher than the air pressure P1 in the first space 52. As a result, since a differential pressure is generated between the first space 52 and the second space 72, the first exhaust valve 60 is closed.

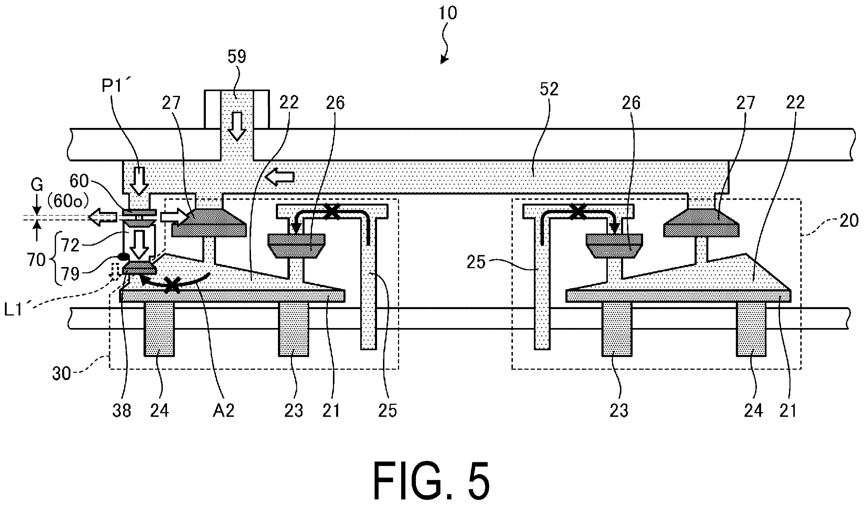

On the other hand, when the pressurization by the first pressurizing unit 30 is stopped, the supply of air to the second space 72 indicated by the arrow A2 is stopped. In this case, as illustrated in FIG. 5, an exhaust state such that the first exhaust valve 60 is opened is achieved. FIG. 5 is a cross-sectional view illustrating an example of the exhaust state of the pressure regulating mechanism in the first embodiment. As illustrated in FIG. 5, while air continues to leak from the second space 72 via the air leakage valve 79 as illustrated by the arrow L1', the air pressure P 1' in the first space 52 is high until the first exhaust valve 60 is opened. Since the volume of the second space 72 is small, the air pressure in the second space 72 rapidly decreases due to the leaking air indicated by the arrow L1'.

As a result, the air pressure in the second space 72 becomes lower than the air pressure in the first space 52. As a result, the closed first exhaust valve 60 is opened, and the first space 52 is in the exhaust state. In this case, a gap G is formed by opening the first exhaust valve 60, and the air in the first space 52 is exhausted from the gap G to the other space by the differential pressure. This makes it possible to rapidly decrease the air pressure in the first space 52 and the cuff 2. The gap G is an example of a communication port.

As described above, the measuring device 1 according to the first embodiment includes the first space 52 for pressurizing the pressurization target 2 and the second space 72 independent of the first space 52. The measuring device 1 includes the first exhaust valve 60 closing or opening the communication port G communicating the first space 52 with the other space. The open/closed state of the first exhaust valve 60 changes according to the pressure difference between the first space 52 and the second space 72. Further, in the first embodiment, the first space 52 is pressurized by the plurality of first pressurizing units 20 and 30, and the second space 72 is pressurized by the first pressurizing unit 30. In such a configuration, the differential pressure between the first space 52 and the second space 72 can be generated with a simple structure without largely changing the configuration of the existing pump. As a result, the structure of the pressure rising type measuring device can be simplified.

### Second Embodiment

In the first embodiment, a configuration has been described such that the first pressurizing unit 30 pressurizing the first space 52 also supplies air to the second space 72, but the embodiment is not limited to this. For example, the second space may be pressurized by another pressurizing unit different from the first pressurizing unit.

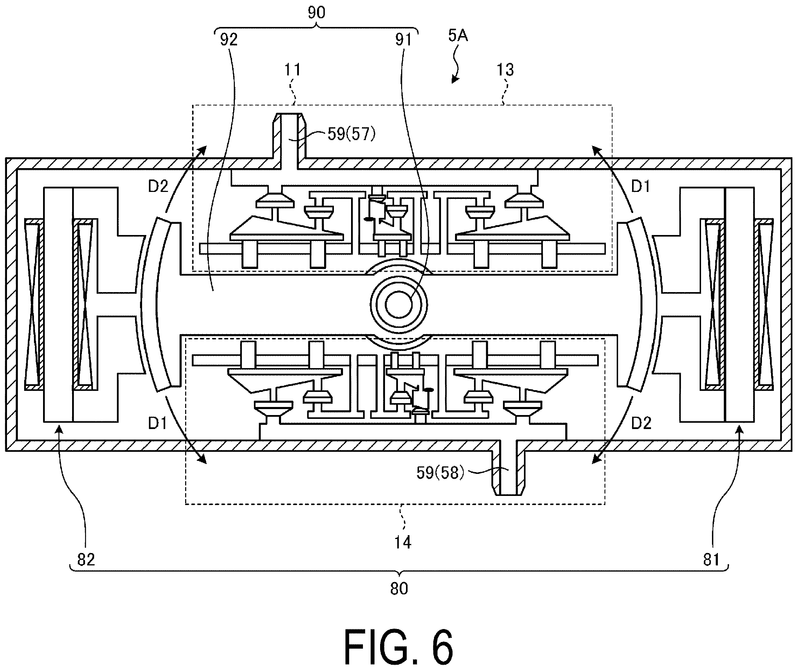

A measuring device 1A according to the second embodiment has the same appearance as the appearance of the measuring device 1 illustrated in FIG. 1 except that the pump unit 5 is changed to a pump unit 5A, and therefore, the illustration is omitted. FIG. 6 is a cross-sectional view illustrating an example of a pump according to the second embodiment. As illustrated in FIG. 6, the pump unit 5A in the second embodiment includes the drive mechanism 80, the swing mechanism 90, pressure regulating mechanisms 13 and 14, and the pair of discharge ports 57 and 58. Hereinafter, the pressure regulating mechanisms 13 and 14 may be referred to as a pressure regulating mechanism 10A when the pressure regulating mechanisms 13 and 14 are not distinguished from each other.

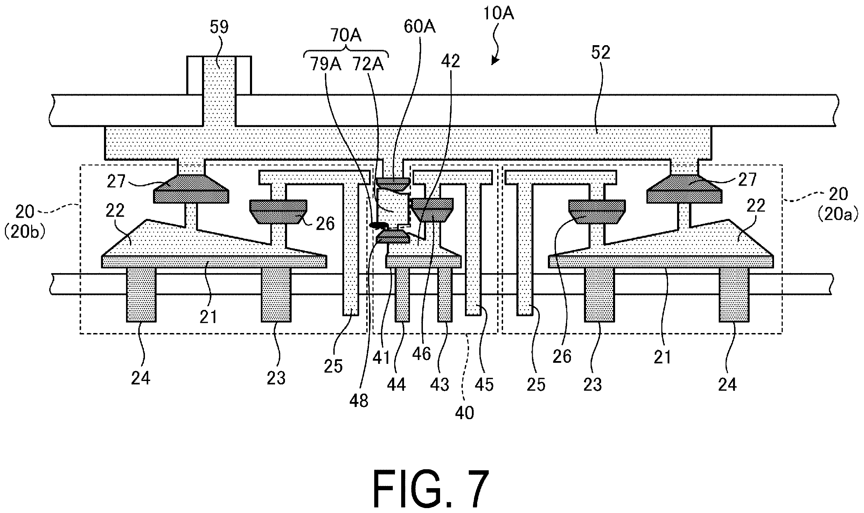

FIG. 7 is a cross-sectional view illustrating an example of the pressure regulating mechanism in the second embodiment. As illustrated in FIG. 7, the pressure regulating mechanism 10A in the second embodiment includes two first pressurizing units 20a and 20b, a second pressurizing unit 40, the first space 52, the first exhaust valve 60, and a pressure regulating unit 70A. Similarly to the first pressurizing units 20 and 30 in the first embodiment, the first pressurizing units 20a and 20b in the second embodiment are disposed at positions facing each other, across the shaft 91 illustrated in FIG. 6, in the direction in which the swing body 92 extends. The first pressurizing units 20a and 20b both have the same configuration as the configuration of the first pressurizing unit 20 in the first embodiment, and thus detailed description will be omitted.

The second pressurizing unit 40 does not supply air to the first space 52, but supplies air to the second space 72A of the pressure regulating unit 70A illustrated in FIG. 7. The second pressurizing unit 40 includes a movable portion 41, a sealed space 42, pressing portions 43 and 44, an intake port 45, an intake valve 46, and a fourth exhaust valve 48.

Similarly to the movable portion 21 and the sealed space 22 of the first pressurizing unit, the movable portion 41 and the sealed space 42 of the second pressurizing unit 40 repeat intake and exhaust in accordance with the up and down movement of the pressing portions 43 and 44. The second pressurizing unit 40 supplies the air supplied from the intake port 45 via the intake valve 46 to the second space 72A via the fourth exhaust valve 48. The intake valve 46 is also, for example, a check valve similar to the intake valve 26.

The fourth exhaust valve 48 is provided between the sealed space 42 and the second space 72A. The fourth exhaust valve 48 is also, for example, a check valve. The fourth exhaust valve 48 allows a flow of air from the sealed space 22 to the second space 72, and restricts a flow of air from the second space 72 to the sealed space 22. As a result, the second pressurizing unit 40 supplies air to the second space 72A without supplying air to the first space 52.

The pressure regulating unit 70A illustrated in FIG. 7 includes the second space 72A and an air leakage valve 79A. The second space 72A is a space independent of the first space 52. The second space 72A is not a sealed space, and air constantly leaks to the other space via the air leakage valve 79A. Since the structure of the pressure regulating unit 70A is the same as the structure of the pressure regulating unit 70 in the first embodiment except that the pressure regulating unit 70A is connected to the second pressurizing unit 40 instead of the first pressurizing unit 30, the detailed description will be omitted.

The second pressurizing unit 40 in the second embodiment may have the same configuration as the first pressurizing unit 20, but in view of the ratio of the volumes of the second space 72A and the first space 52, for example, the volume of the sealed space or the displacement of the movable wall of the second pressurizing unit 40 may be smaller than the volume or the displacement of the first pressurizing unit 20, or the number of the second pressurizing unit 40 may be smaller than the number of the first pressurizing unit 20. For example, in the second embodiment, as illustrated in FIGS. 6 and 7, two second pressurizing units 40 are provided for four first pressurizing units 20 in the pump unit 5A. In the example illustrated in FIG. 7, the second pressurizing unit 40 and the pressure regulating unit 70A are disposed between the two first pressurizing units 20a and 20b in the direction in which the swing body 92 extends, but the positions where the second pressurizing unit 40 and the pressure regulating unit 70A are disposed are not limited to this.

The first exhaust valve 60A in the second embodiment is provided between the first space 52 and the second space 72A. Similarly to the first exhaust valve 60, for example, the open/closed state of the first exhaust valve 60A changes according to the air pressure difference (differential pressure) between the first space 52 and the second space 72.

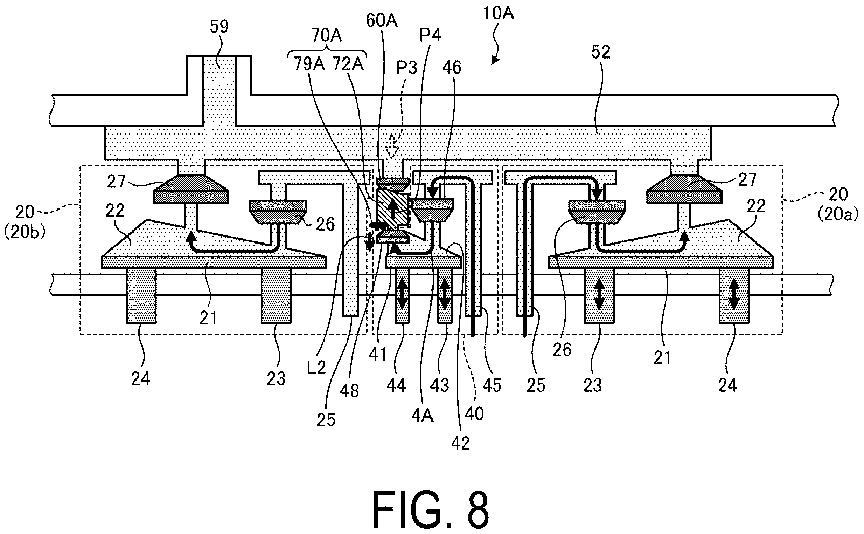

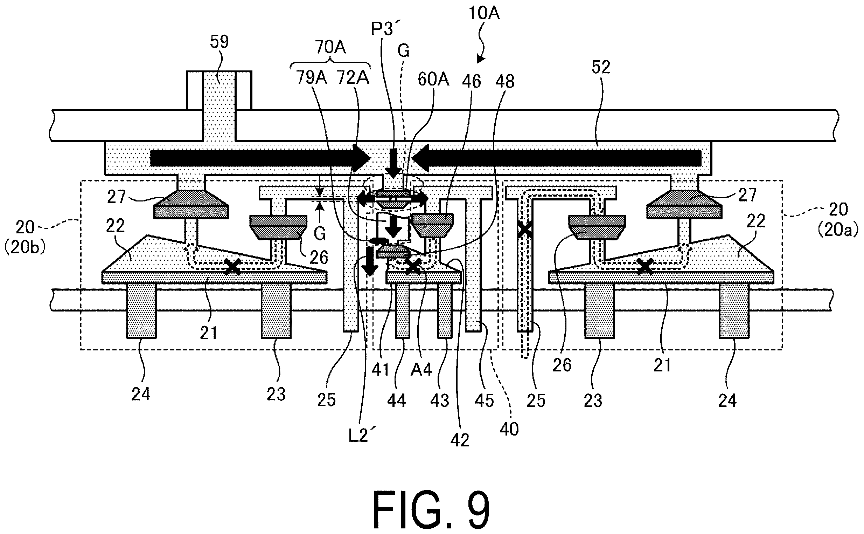

The intake state and the exhaust state in the second embodiment will be described with reference to FIGS. 8 and 9. FIG. 8 is a cross-sectional view illustrating an example of an intake state in the pressure regulating mechanism according to the second embodiment. As illustrated in FIG. 8, in the intake state, the amount of the air A4 supplied from the second pressurizing unit 40 to the second space 72A is larger than the amount of the air L2 leaking from the air leakage valve 79. In this case, when the second pressurizing unit 40 starts pressurization, the air pressure P4 in the second space 72 rapidly increases compared to the increase in the air pressure P3 in the first space 52. As a result, since a differential pressure is generated between the first space 52 and the second space 72A, the first exhaust valve 60A is closed.

On the other hand, when the pressurization by the second pressurizing unit 40 is stopped, the supply of air to the second space 72A is stopped. FIG. 9 is a cross-sectional view illustrating an example of an exhaust state of the pressure regulating mechanism in the second embodiment. As illustrated in FIG. 9, while air continues to leak from the second space 72A via the air leakage valve 79A as illustrated by the arrow L2', the air pressure P3' in the first space 52 is high until the first exhaust valve 60A is opened. As a result, the air pressure in the second space 72A becomes lower than the air pressure in the first space 52. As a result, a gap G is formed by opening the closed first exhaust valve 60, and the air in the first space 52 is exhausted from the gap G to the other space by the differential pressure.

As described above, the measuring device 1A in the second embodiment includes the first pressurizing units 20a and 20b pressurizing the first space 52 and the second pressurizing unit 40 pressurizing the second space 72. According to such a configuration, it is possible to easily generate a differential pressure between the first space 52 and the second space 72A by the second pressurizing unit 40 pressurizing only the second space 72A.

### Modifications

The configuration of each of the embodiments has been described above, but the embodiments are not limited to these configurations. For example, a plurality of the second spaces 72 may be provided for one first space 52, and a plurality of the pressurizing units pressurizing the second spaces 72 may be provided. For example, two or more first pressurizing units 30 or two or more second pressurizing units 40 may be provided, or both the first pressurizing unit 30 and the second pressurizing unit 40 may be provided.

In addition, although a configuration such that the air leakage valve 79 is provided in the pressure regulating unit has been described, the present invention is not limited to this configuration as long as the air in the second space 72 can be constantly leaked to the other space. For example, the surface of the second space 72 may be roughened or grooved, or fine holes may be provided in the second space 72. Further, a configuration such that air leaks from the second exhaust valve 38 or the fourth exhaust valve 48 may be adopted.

In addition, for example, the flow path load from the first pressurizing unit 20 or 30 to the first space 52 may be larger than the flow path load from the first pressurizing unit 30 to the second space 72 or the flow path load from the second pressurizing unit 40 to the second space 72A. Accordingly, even when there is no volume difference between the first space and the second space, a differential pressure between the first space and the second space can be generated. In order to change the flow path load, for example, the hardness of the valve may be changed between the third exhaust valve 27 and the second exhaust valve 38 or the fourth exhaust valve 48, or the length or thickness of the flow path may be changed between the flow path to the first space 52 and the flow path to the second space 72. In addition, it is possible to more easily generate the differential pressure by combining the difference in the flow path load and the difference in the volume.

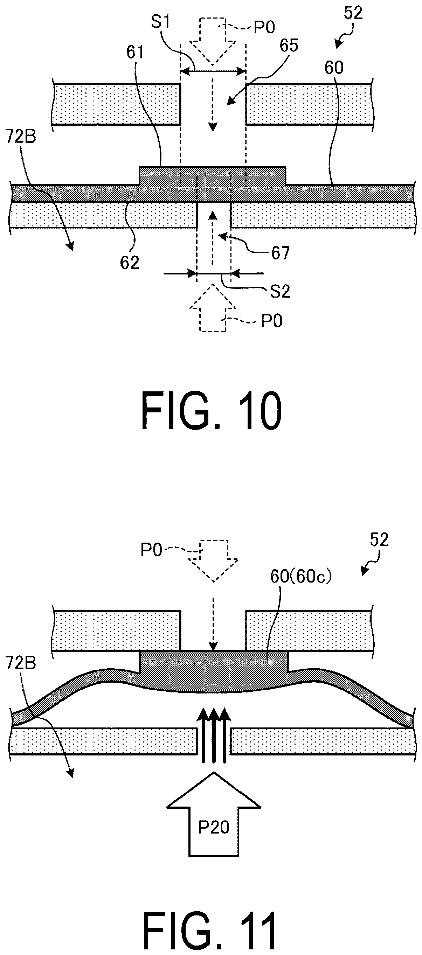

Further, a configuration such that the first exhaust valve is in the open state may be adopted even in a state such that the differential pressure is small or in a state such that no differential pressure is generated. For example, in the first embodiment, the pressure receiving area of the first surface 61 of the first exhaust valve 60 facing the first space 52 may be different from the pressure receiving area of the second surface 62 of the first exhaust valve 60 facing the second space 72. FIG. 10 is an enlarged cross-sectional view illustrating an example of the positional relationship between the first exhaust valve and an opening in a first modification. Although the following description will be made based on the configuration of the first embodiment, each modification can also be applied to the first exhaust valve 60A, the first space 52, and the second space 72A in the second embodiment.

As illustrated in FIG. 10, the first surface 61 of the first exhaust valve 60 faces the first space 52 via an opening 65. Further, the second surface 62 of the first exhaust valve 60 faces a second space 72B via the opening 67. In the first modification, the area S2 of the opening 67, that is, the pressure receiving area of the second surface 62 with respect to the second space 72B, is smaller than the area S1 of the opening 65, that is, the pressure receiving area of the first surface 61 with respect to the first space 52. FIG. 10 illustrates a state such that the first space 52 and the second space 72B are neither pressurized nor depressurized. In such a state, the air pressure P0 in the first space 52 and the second space 72B is substantially the same as the air pressure in the other space.

Operations during pressurization and depressurization in such a configuration will be described with reference to FIGS. 11 to 13. FIG. 11 is an enlarged cross-sectional view illustrating an example of the operation of the first exhaust valve in the intake state in the first modification. As illustrated in FIG. 11, when the air pressure P20 in the second space 72B becomes sufficiently higher than the air pressure P0 in the first space 52, the first exhaust valve 60 is in the closed state. Then, when the first space 52 is closed by the first exhaust valve 60c in the closed state, air is supplied from the first pressurizing units 20 and 30 to the first space 52, so that the air pressure in the first space 52 also increases.

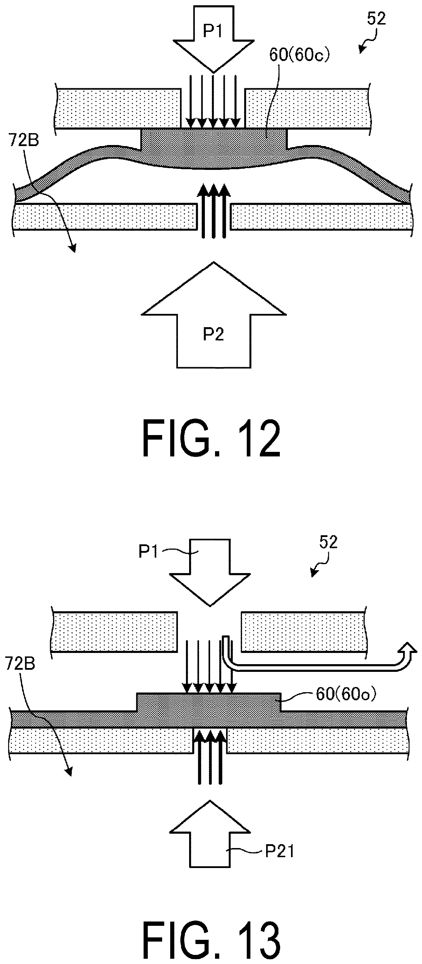

FIG. 12 is an enlarged cross-sectional view illustrating another example of the operation of the first exhaust valve in the intake state in the first modification. As illustrated in FIG. 12, even when the air pressure of the first space 52 increases from P0 to P1, if the air pressure P2 in the second space 72B is sufficiently higher than the air pressure P1, the first exhaust valve 60 maintains the closed state.

Next, when the supply of air by the first pressurizing units 20 and 30 is stopped, the air pressure of the second space 72B rapidly decreases due to air leakage from the air leakage valve 79 illustrated in FIG. 3. On the other hand, since the first exhaust valve 60 is in the closed state, the air pressure P1 of the first space 52 does not immediately decrease even when the supply of air is stopped. Accordingly, as illustrated in FIG. 13, the first exhaust valve 60 transitions to the open state in accordance with the difference between the air pressure P1 of the first space 52 and the air pressure P21 of the second space 72B. As a result, the air in the first space 52 is exhausted to the other space. FIG. 13 is an enlarged cross-sectional view illustrating an example of the operation of the first exhaust valve in the exhaust state in the first modification. In FIG. 13, the air pressure P21 in the second space 72B is substantially the same as the air pressure P1 in the first space 52. That is, FIG. 13 illustrates a state before the air pressure in the second space 72B is sufficiently reduced as compared with the exhaust state illustrated in FIG. 5.

In the first modification, since the pressure receiving area S1 of the first exhaust valve 60 with respect to the first space 52 is larger than the pressure receiving area S2 with respect to the second space 72B, the first exhaust valve 60 transitions to the open state before the air pressure P21 of the second space 72B becomes lower than the air pressure P1 of the first space 52. According to such a configuration, the first space 52 can be more quickly shifted to the exhaust state.

For example, the pressure receiving areas S1 and S2 of the first exhaust valve 60 may be set such that the first exhaust valve 60 can transition to the open state even when the air pressure of the second space is higher than the air pressure P1 of the first space. In such a configuration, it is possible to transition to the exhaust state more quickly than in the first modification, and it is possible to stably continue the exhaust state without the first exhaust valve 60 returning to the closed state even in a case where the decrease in the air pressure of the second space is gradual in the exhaust state.

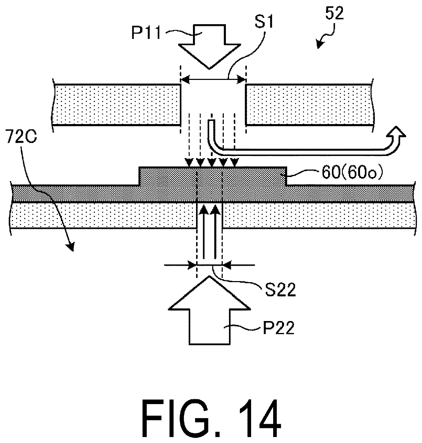

FIG. 14 is an enlarged cross-sectional view illustrating another example of the operation of the first exhaust valve in the exhaust state in a second modification. As illustrated in FIG. 14, the pressure receiving area S22 in the second modification is smaller than the pressure receiving area S2 in the first modification. FIG. 14 illustrates a state such that the exhaust from the first space 52 progresses and the air pressure decreases from P1 to P 11. In the second modification, the air pressure in the first space 52 decreases more rapidly than the air pressure in a second space 72C. In this case, as illustrated in FIG. 14, the air pressure P11 of the first space 52 may be lower than the air pressure P22 of the second space 72C.

Even in such a state, in the second modification, due to the difference between the pressure receiving area S1 with respect to the first space 52 and the pressure receiving area S22 with respect to the second space 72C, the first exhaust valve 60 is suppressed from transitioning to the open state or vibrating between the open state and the closed state. Thus, a stable exhaust state can be continued.

In each of the modifications, the air pressure difference between the first space 52 and the second space required for the first exhaust valve 60 to transition from the open state to the closed state is larger than the air pressure difference in each of the embodiments. However, in each of the modifications, since the volumes of the second spaces 72B and 72C are sufficiently smaller than the volume of the first space 52, the influence on the time lag at the time of pressurizing the second space is small.

The embodiment and the modifications of the present invention have been described, but the present invention is not limited to the embodiment and the modifications but can be variously modified without departing from the spirit of the present invention. Various modifications within a scope not departing from the gist are included in the technical scope of the present invention, and this is obvious to a person having skill in the art from the description of the claims.

### Reference Signs List

1, 1A Measuring device (blood pressure monitor), 2 Cuff, 3 Main body, 4 Tube, 5, 5A Pump unit, 6 Control unit, 10, 10A (11 to 14) Pressure regulating mechanism, 20, 20a, 20b, 30 First pressurizing unit, 21, 41 Movable portion, 22, 42 Sealed space, 23, 24, 43, 44 Pressing portion, 25, 45 Intake port, 26, 46 Intake valve, 27 Third exhaust valve, 38 Second exhaust valve, 40 Second pressurizing unit, 48 Fourth exhaust valve, 52 First space, 57, 58, 59 Discharge port, 60, 60A First exhaust valve, 70, 70A Pressure regulating unit, 72, 72A, 72B, 72C Second space, 79, 79A Air leakage valve, 80 Drive mechanism, 81, 82 Actuator, 90 Swing mechanism, 91 Shaft, 92 Swing body

## Claims

1. A measuring device comprising:
a first space for pressurizing a pressurization target;
a second space independent of the first space; and
a first exhaust valve configured to close or open a communication port communicating the first space with another space, wherein
an open/closed state of the first exhaust valve changes in accordance with a pressure difference between the first space and the second space.

2. The measuring device according to claim 1, wherein
when a pressure in the first space is lower than a pressure in the second space, the first exhaust valve is closed and the first space pressurizes the pressurization target, and
when the pressure in the first space is equal to or higher than the pressure in the second space, the first exhaust valve is opened to reduce the pressure in the first space.

3. The measuring device according to claim 1 or 2, wherein
a volume of the first space is larger than a volume of the second space.

4. The measuring device according to claim 1 or 2, wherein
a flow path load of a suction port of the first space is larger than a flow path load of a suction port of the second space.

5. The measuring device according to any one of claims 1 to 4, further comprising:
a second exhaust valve provided in the suction port to the second space; and
an air leakage valve configured to communicate the second space with another space.

6. The measuring device according to any one of claims 1 to 5, further comprising a first pressurizing unit configured to pressurize the first space and the second space.

7. The measuring device according to claim 6, wherein
the first space is pressurized by a plurality of the first pressurizing units, and
the second space is pressurized by at least one of the first pressurizing units.

8. The measuring device according to any one of claims 1 to 5, further comprising:
a first pressurizing unit configured to pressurize the first space; and
a second pressurizing unit configured to pressurize the second space.

9. The measuring device according to any one of claims 1 to 8, wherein
the first exhaust valve includes a first surface facing the first space and a second surface facing the second space, and
a pressure receiving area of the first surface is larger than a pressure receiving area of the second surface.
